# EUROPEAN PATENT SPECIFICATION

(11) **EP 2 125 761 B1**
(45) Date of publication and mention of the grant of the patent: **01.03.2017**
(21) Application number: 08705438.3
(22) Date of filing: 07.02.2008
(51) Int. Cl.: C07D 277/82

(54) **NOVEL PROCESS FOR SYNTHESIS OF PRAMIPEXOLE AND ITS PHARMACEUTICALLY ACCEPTABLE SALTS**
NEUARTIGES VERFAHREN ZUR SYNTHESE VON PRAMIPEXOL UND SEINEN PHARMAZEUTISCH AKZEPTABLEN SALZEN
NOUVEAU PROCÉDÉ POUR UNE SYNTHÈSE DE PRAMIPEXOLE ET DE SES SELS ACCEPTABLES DU POINT DE VUE PHARMACEUTIQUE

(30) Priority: 07.02.2007 SI 200700029
(43) Date of publication of application: 02.12.2009
(73) Proprietor: KRKA, tovarna zdravil, d.d., Novo mesto, 8501 Novo mesto (SI)
(72) Inventor: ZIVEC, Matej, 6000 Koper (SI); GOBEC, Stanislav, 1000 Ljubljana (SI); ANZIC, Borut, 1236 Trzin (SI); ZUPET, Rok, 1000 Ljubljana (SI); KOLENC, Ivanka, 8000 Novo Mesto (SI)
(74) Representative: Redensek, Vladimira
(86) International application number: PCT/SI2008/000007
(87) International publication number: WO 2008/097203

(56) References cited:
- EP-A- 0 186 087
- DATABASE CA [Online] CHEMICAL ABSTRACTS SERVICE, COLUMBUS, OHIO, US; BALICKI, ROMAN ET AL: "New method for preparing pramipexole dihydrochloride monohydrate" XP002483830 retrieved from STN Database accession no. 2006:504297 & PRZEMYSL CHEMICZNY , 85(5), 344-346 CODEN: PRCHAB; ISSN: 0033-2496, 2006,

## Description

### Technical Field of the Invention

The present invention belongs to the field of organic chemistry and relates to a novel process of synthesis of pramipexole and its pharmaceutically acceptable salts. Pramipexole, preferably its dihidrochloride monohydrate, is a compound used for a treatment of Parkinson's disease.

### Technical Problem

There existed a need for a novel economical process which would provide high yields and, at the same time, be industrially acceptable.

### Prior Art

Pramipexole, with a chemical name S(-)-2-amino-4,5,6,7-tetrahydro-6-propylaminobenzothiazole, belongs to a family of bioisosters of hydroxytetraline and ergoline, which have been developed as antagonists of dopamine receptors. Pramipexole is used for the treatment of signs and symptoms of Parkinson's disease and restless leg syndrome.

A new synthetic approach has been published in Przemysl Chemiczny, 85/5 (2006), Balicki Roman; Ciesielska Agnieszka, 344-346.

Pramipexole is the subject of patent EP 186087, describing several possible processes for synthesis
a) a reaction of cyclohexanone of formula 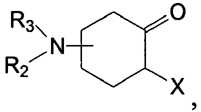 wherein X stands for halogen, with thiourea of formula 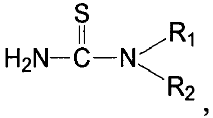 wherein R₁ in R₂ can stand for H, alkyl, allyl and other groups;
b) a reaction of cyclohexanone of formula 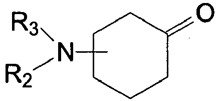 with formamidine disulfide;
c) a cleavage of a protecting group from the compound of formula 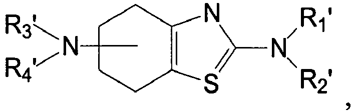 wherein at least one of R₁', R₂', R₃', R₄' represents amino protecting group;
d) a reduction of acyl or phenylacyl group with the use of metal hydride in a solvent, whereby an appropriate alkyl or phenylalkyl group is obtained;
e) a reaction of a compound of formula 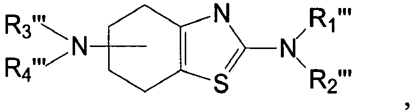 wherein at least one of the substituents represents hydrogen atom, with a compound of formula R₅-Z, wherein R₅ stands for alkyl and Z stands for a nucleophilically exchangeable group.

There also exists a series of patents, describing resolution of racemic pramipexole, the examples are in EP 1318987, wherein a resolution of diastereoisomeric salts by crystallization is described, in WO 2006/003471, wherein a resolution with chiral chromatography is described, in WO 2006/012276, wherein resolution with liquid chromatography is described, in WO 2006/120268, wherein separation with derivatives of tartaric acid is described.

The processes for the preparation of pramipexole with reductive amination of diamine intermediate of formula 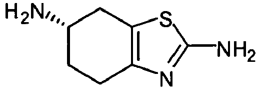 are described in WO 2005/092871, WO 2006/003677, WO 2006/070349, WO 2006/097014.

The process according to the present invention represents novel inventive approach to the solution of a synthesis, providing high conversion rate of intermediates and a process, whereby a formation of undesired side products is avoided. An advantage of the process according to the present invention is that high optical purity is preserved in all steps and that racemization does not occur in any of the steps.

### Description of the Invention

The subject of the present invention is a process for the preparation of pramipexole, comprising a process for synthesis, shown in the scheme 1

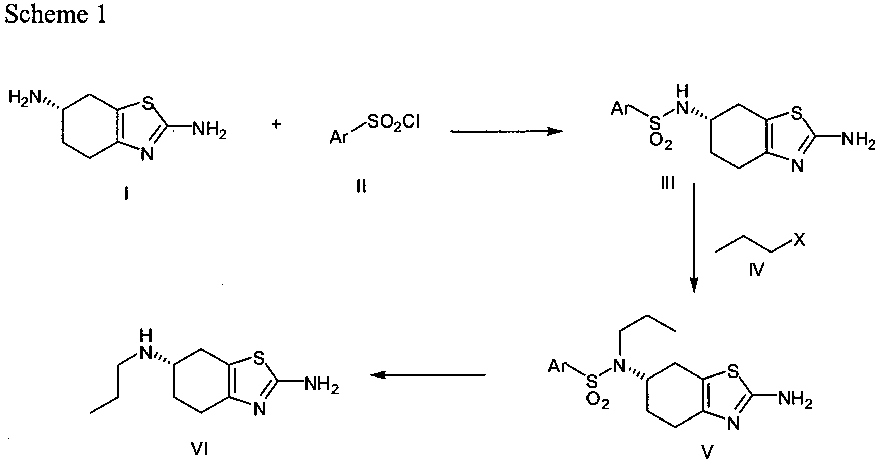

A process according to the present invention comprises:
(i) a reaction of 4,5,6,7-tetrahydrobenzo[*d*]thiazole-2,6-diamine of formula I with nitro or halogen substituted or unsubstituted aryl sulfonyl chloride of formula II, whereby sulfonamide intermediate of formula III is obtained;
(ii) a reaction of an intermediate of formula III with a compound of formula IV, wherein X can be selected from Cl, Br, I, or other nucleophilically exchangeable group, such as OTs, OMs, whereby an intermediate of formula V is obtained;
(iii) a conversion of intermediate of formula V with a selective reagent for a cleaving off of the sulfonyl protection into pramipexole of formula VI;
(iv) optionally, pramipexole can be converted into pharmaceutically acceptable salt.

The process can be carried out continuously or uncontinuously.

A reaction of a compound I with a compound II is carried out in the presence of an inorganic or organic base in an organic solvent. Organic base can be selected from a group of tertiary amines, such as trimethylamine, triethylamine, diisopropylethyl amine, tri-n-propylamine, tri-n-butylamine, N,N,N',N'-tetramethyl-1,3-propanediamine, N-methyl morpholine, N,N'-dimethylpiperazine, 1,4-diazabicyclo[2.2.2]octane, 1,5-diazabicyclo[4.3.0]-5-nonene; 1,8-diazabicyclo[5.4.0]-7-undecene; heterocyclic aromatic amines selected from pyridine, pyrrolidine such as N-methyl pyrrolidine, dimethylpyrrolidine, 4-dimethylamino pyridine, colidine, lutidine; preferably triethyl amine. Inorganic base can be selected from alkaline and alkaline earth hydroxides, such as NaOH, KOH, LiOH, Mg(OH)₂, carbonates, hydrogen carbonates, quarternary ammonium hydroxides, such as tetrabutyl ammonium hydroxide, tetraethylammonium hydroxide, or alkoxides.

An organic solvent can be selected from ketones such as acetone, methylethyl ketone; ethers such as THF, diethylether; nitriles such as acetonitrile; amides such as dimethyl acetamide, dimethyl formamide; pyrrolidones such as n-methylpyrrolidone; halogenated alkanes such as methylene chloride, chloroform.

Preferably the reaction is carried out in such a manner that nitro-substituted aryl sulfonyl chloride is dissolved in a solvent, cooled to a temperature from -20°C to 10°C, preferably under 0°C, a base and then the compound of formula I are added and it is allowed that the reaction temperature gradually rises to room temperature.

As a compound of formula II aryl sulfonyl chloride can be used, preferably nitro or halogen substituted aryl sulfonyl chloride.

As nitro substituted aryl sulfonyl chloride, a mono-, di- or tri-nitro substituted benzene sulfonyl chloride such as 2-nitrobenzene sulfonyl chloride, 4-nitrobenzene sulfonyl chloride 2,4-, 2,3-, 2,5-, 2,6-dinitrobenzene sulfonyl chloride, 2,4,6-trinitrobenzene sulfonyl chloride, preferably 2-nitrobenzene sulfonyl chloride can be used. As halogen substituted aryl sulfonyl chlorides, 4-Br-, 4-Cl- and 4-F-benzene sulfonyl chlorides can be used.

The isolation of intermediate of formula III is carried in such a manner that, after desalination a solution is evaporated to a dry residue, which is poured over with water and, under stirring, a yellow precipitate falls out, which can be optionally washed with water. Alternatively the solution could be evaporated to approximately 1/3 of the volume and water could be added.

A reaction of intermediate III with intermediate IV is carried out in an organic solvent in the presence of a base, optionally in a presence of a catalyst such as KI, whereby an intermediate V is obtained.

As a base an organic or inorganic base can be used. As organic bases primary, secondary and tertiary amines such as triethylamine, diisopropylamine, methylamine, pyridine, pyrrolidine, piperazine, pyrimidine can be used. Inorganic base can be selected from alkaline or alkaline earth hydroxides such as NaOH, KOH, LiOH, Mg(OH)₂, carbonates such as K₂CO₃, Cs₂CO₃, hydrogen carbonates, quaternary ammonium hydroxides such as tetrabutyl ammonium hydroxide, tetraethylammonium hydoxide or alkoxides, preferably potassium carbonate, cesium carbonate.

As a solvent ketons such as acetone, methylethylketone diethylketone; ethers such as THF, diethyl ether, dioxane; nitriles such as acetonitrile, propionitrile; alcohols such as propanol; amides such as dimethyl acetamide, dimethyl formamide; pyrrolidones such as N-methylpyrrolidone, aromatic hydrocarbons such as toluene, xylene; sulfoxides such as dimethyl sulfoxide can be used.

As a reagent of formula IV propyl bromide, propyl iodide, propyl chloride, propyl tosylate, propyl methanesulfonate, propyl sulfate, preferably propyl bromide can be used.

An isolation of intermediate V is carried out in such a manner that a reaction mixture is desalinated, the solution is evaporated to dryness and the residue is dissolved in dichloromethane. This solution is washed with alkaline aqueous solution, saturated aqueous NaCl solution and dried with Na₂SO₄. After the evaporation an oily product is obtained.

The obtained intermediate V, is converted into pramipexole by the use of a reagent for a selective removal of arylsulfonyl group. As a reagent for selective removal of nitrobenzene sulfonyl group a thiol in the presence of a base or alkoxide is used. As a thiol there can be used thiophenol, thioglycolic acid, mercaptoethanol, preferably thioglycolic acid. As a base an organic or inorganic base can be used. As organic bases there can be used primary, secondary and tertiary amines, such as trimethylamine, triethylamine, diisopropylethylamine, tri-n-propylamine, tri-n-butylamine, N,N,N',N'-tetramethyl-1,3-propanediamine, N-methyl morpholine, N,N'-dimethylpiperazine, 1,4-diazabicyclo[2.2.2]octane, 1,5-diazabicyclo[4.3.0]-5-nonene; 1,8-diazabicyclo[5.4.0]-7-undecene; heterocyclic aromatic amines, selected from pyridine, pyrrolidine such as N-methylpyrrolidine, dimethylpyrrolidine, 4-dimethylaminopyridine, colidine, lutidine; preferably triethylamine. As inorganic bases there can be used hydroxides, preferably lithium hydroxide, carbonates, hydrogen carbonates, alkoxides of alkaline or alkaline earth metals, preferably potassium carbonate, cesium carbonate. As an alkoxide there can be used alkoxides such as methoxides, ethoxides, propoxides, preferably methoxides, of alkaline or alkaline earth metals.

The reaction can be carried out in an organic solvent such as ketones, such as acetone, methylethylketone, diethylketone; ethers such as THF, diethylether, methylethylether, dioxane, dimethoxyethane; nitriles such as acetonitrile, propionitrile; amides such as dimethyl acetamide, dimethyl formamide; pyrrolidones such as N-methylpyrrolidone, aromatic hydrocarbons such as toluene, xylene, sulfoxides such as dimethylsulfoxide, halogenated alkanes.

The reaction runs at a temperature from room temperature to reflux temperature of a reaction mixture, preferably at a temperature from 40-70°C.

Preferable embodiment of the reaction according to the present process is that a reagent for removal of the protection is previously activated in such a manner that it is stirred together with a base from 10 minutes to 1 hour, preferably for 30 minutes, prior to addition of the intermediate of formula V.

As the starting compound I a product, obtained according to any of the known processes from e.g. EP 186087, WO 2004/041797, EP 1731514, can be used. The starting compound may be obtained also according to the manufacturing processes disclosed in CN 1772744, J Med Chem, 1987, vol.30, no.3, p.494-498, J Het Chem, 2000, vol.37, no.2, p.405-409.

The reaction is usually being followed by the use of analytic methods such as thin layer chromathography, HPLC, gas chromatography and similar, and when the absence of the starting compound is confirmed, the reaction is completed.

An isolation is carried out in such a manner that, to a mixture of a free base of pramipexole and a solvent, HCl in a form of gas and a solution of HCl gas in a solvent respectively is added. Pramipexole crystallizes from a solution in a form of dihydrochloride hydrate. Solvents, suitable for isolation, are alcohols, preferably methanol, ethanol, isopropanol, 2-butanol, esters, preferably methylacetate, ethylacetate, ketones, preferably acetone, t-butyl methyl ketone. Isolation of pramipexole in a form of dihydrochloride can be carried out with re-precipitation with anti-solvents. The product can be isolated by lyophilization or fast evaporation of aqueous solutions of pramipexole dihydrochloride.

Pramipexole in the form of dihydrochloride hydrate is recrystallized from alcohols or a mixture of alcohols, preferably from methanol with addition of ethanol.

Pramipexole base, obtained according to the process of the present invention, can further be converted into dihydrochloride according to processes described in EP 186087, WO 2006/070349, EP1318987, WO 2006/012276.

A compound, obtained according to the process, which is the object of the present invention, can be used as a therapeutic active ingredient, which, together with a pharmaceutically acceptable carrier, can be used as a medicine. It can be used for the treatment of symptoms associated with Parkinson's disease as well as for the treatment of restless leg syndrome.

The compound, obtained according to the present invention, can be incorporated into a pharmaceutical formulation, which, in addition to the active ingredient, also comprises pharmaceutically acceptable adjuvants, generally known to an average person skilled in the art of pharmacy as described in e.g. EP 1318987, WO 2006/015944, WO 2006/015942, WO 03/053402, IPCOM000145762D. In this formulations the average particle size of the active ingredient could be from 5 to 500 microns, preferably from 10 to 300 microns, most preferably from 50-170 microns. Size distribution of particles can be determined by laser light scattering method using e.g. a Malvern Mastersizer 2000 Apparatus with Isopar L as the dilution medium.

The pharmaceutical formulation can be prepared according to the processes, known to an average person skilled in the art of pharmacy, such as direct compacting, dry granulation or wet granulation.

Reactions can be followed and purity of the products can be controlled by the use of the following two methods:

### a) Chromatographic purity

High performance liquid chromatography, HPLC, percentage method

| | | | |
|---|---|---|---|
| **Column:** | | Gemini C-18 110A 150 mm x 4.6 mm, 5 µm particles (alternatively | |
| | | Alltima C-18, 250 mm x 4.6 mm, 5 µm particles could be used) | |
| **Mobile phase:** | | | |
| | *- buffer solution A:* | | 0.01M KH₂PO₄ (pH=10) |
| | *- buffer solution B:* | | CH₃CN : CH₃OH : H₂O = 600 : 300: 100 (pH=10) |
| | - gradient: | | |

| **Time (min)** | **% A** | **% B** |
|---|---|---|
| 0.0 | 80 | 20 |
| 18 | 50 | 50 |
| 24 | 30 | 70 |
| 40 | 30 | 70 |
| 41 | 80 | 20 |
| **post time** | **7 min** | |

| | |
|---|---|
| **Flow:** | approximately 1.0 mL/min |
| **Detection:** | UV, wave length: 263 nm |
| **Injection:** | 20 µL |
| **Temperature:** | 25 °C (room temperature) |

### b) Enantiomeric purity

High performance liquid chromatography, HPLC.

**Evaluation method: area %**

| | |
|---|---|
| **Column:** | Chiralpack IA 250 mm x 4.6 mm, 5 µm particles |
| **Mobile phase:** | hexane/ethanol/DEA in a ratio of 75/25/0.05 |
| **Column temperature:** | 25°C |
| **Flow:** | 0,8 mL/min |
| **Detection:** | UV, 263nm |
| **Injection:** | 20 µL |

Alternatively the following chromatographic conditions could be used:

| | |
|---|---|
| **Column:** | Chiralpack AD-H 250 mm x 4.6 mm, 5 µm particles |
| **Mobile phase:** | ethanol/hexane/DEA in a ratio of 90/10/0.1 |
| **Column temperature:** | 25°C |
| **Flow:** | 0,5 mL/min |
| **Detection:** | UV, 263nm |
| **Injection:** | 5 µL |

The present invention is illustrated with the following examples without being limited thereto.

### Examples

### Example 1

### Synthesis of N-(2-amino-4,5,6,7-tetrahydrobenzo[d]thiazole-6-yl)-2-nitrobenzenesulfonamide

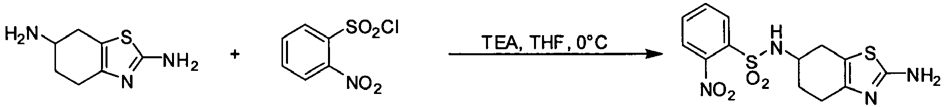

o-Nitrobenzenesulfonyl chloride (8.865 g, 40 mmol) was dissolved in 100 ml of THF and during stirring cooled to -10°C (ice + salt). Then, first 3 equiv. of triethylamine (Et₃N) (120 mmol, 16.8 ml) and then also 1.1 equiv. of 4,5,6,7-tetrahydrobenzo[*d*]thiazol-2,6-diamine (7.605 g, 45 mmol) were added. The formed suspension was, during stirring, gradually heated to room temperature and was left standing until the reaction was completed. In the course of the reaction, in addition to a soluble product, also in THF non-soluble Et₃NH⁺Cl⁻ was formed, which was, at the end of the reaction, filtered off by suction and the reaction mixture was evaporated to dryness on a rotatory evaporator. The residue was poured over with H₂O (300 ml), whereby on the bottom of a round-bottom flask an orange viscous liquid was obtained. After rubbing with the glass stick a yellow precipitate (*N*-(2-amino-4,5,6,7-tetrahydrobenzo[*d*]thiazole-6-yl)-2-nitrobenzenesulfonamide) was formed. The precipitate was filtered off and washed with 100 ml of cold ethylether and dried. The yield of the reaction was 95%.

### Example 2

### Synthesis of N-(2-amino-4,5,6,7-tetrahydrobenzo[d]thiazole-6-yl)-2-nitro-N-propylbenzenesulfonamide

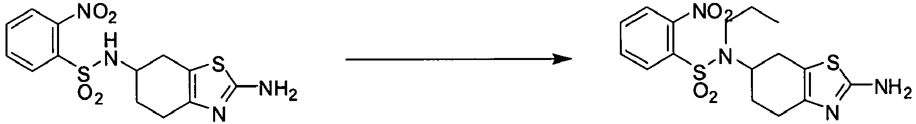

### Process A:

*N-*(2-amino-4,5,6,7-tetrahydrobenzo[*d*]thiazole-6-yl)-2-nitrobenzenesulfonamide (1.770 g, 5 mmol) and K₂CO₃ (2.856 g, 20 mmol) were suspended in acetonitrile (40 ml) and was, during stirring, heated to 60°C. Then propylbromide (1.65 ml, 18 mmol) was added and the reaction was left to run over night (the course of the reaction was followed by the use of a suitable method).

After the reaction was completed, the present precipitate was filtered off by suction. The reaction mixture was evaporated to dryness and the residue was dissolved in dichloromethane (150 ml). Organic phase was washed with 1M NaOH (3 x 50 ml), saturated NaCl solution (2 x 50 ml) and dried with Na₂SO₄. After evaporation of dichloromethane an orange oily product *N*-(2-amino-4,5,6,7-tetrahydrobenzo[d]thiazole-6-yl)-2-nitro-N-propylbenzenesulfonamide was obtained.

### Process B:

*N*-(2-amino-4,5,6,7-tetrahydrobenzo[*d*]thiazole-6-yl)-2-nitrobenzenesulfonamide (1.770 g, 5 mmol), Cs₂CO₃ (3.909 g, 12 mmol) and KI (0.415 g, 2.5 mmol) was suspended in acetonitrile (40 ml) and heated to 60°C. Then propyl bromide (0.9 ml, 10 mmol) was added and the course of the reaction was followed by the use of a suitable method.

The process of the isolation was the same as in the process A.

### Example 3

### Synthesis of N⁶-propyl-4,5,6,7-tetrahydrobenzo[d]thiazole-2,6-diamine

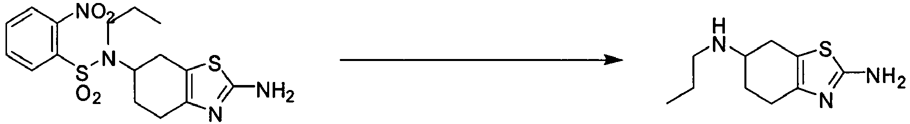

### Process A:

K₂CO₃ (2.073 g, 15 mmol) was suspended in 20 ml of DMF (stored above molecular sieves), thioglycolic acid (SHCH₂COOH, 0.6 ml, 7.5 mmol) was added and stirred for 30 minutes. Then *N*-(2-amino-4,5,6,7-tetrahydrobenzo[*d*]thiazole-6-yl)-2-nitro-N-propylbenzenesulfonamide (0,99 g, 2,5 mmol), dissolved in 20 ml of DMF was added and the reaction was left to run over night. After the reaction was completed, DMF was evaporated, the residue was poured over with H₂O (100 ml) and 1M NaOH (200 ml). The aqueous phase was then washed with dichloromethane (3 x 80 ml) and the combined organic fractions were dried with z Na₂SO₄. After the evaporation of the solvent an oily residue of orange-red colour (presence of DMF is possible) was obtained.

### Process B:

LiOH (0.5 g, 20 mmol) was suspended in 20 ml of DMF (stored above molecular sieves), thioglycolic acid (SHCH₂COOH, 0.6 ml, 7.5 mmol) was added and stirred for 30 minutes. Then *N*-(2-amino-4,5,6,7-tetrahidrobenzo[*d*]thiazole-6-yl)-2-nitro-N-propylbenzenesulfonamide (0.99 g, 2.5 mmol), dissolved in 20 ml of DMF was added and the reaction was left to run over night (the solution was coloured in orange-red).

After the reaction was completed, DMF was evaporated off, the residue was poured over with H₂O (100 ml) and 1M NaOH (200 ml). The aqueous phase was then washed with dichloromethane (3 x 80 ml) and the combined organic fractions were dried with Na₂SO₄. After the evaporation of the solvent an oily residue of an orange-red colour was obtained.

### Example 4

### Pramipexole dihydrochloride monohydrate

(*S*)-(-)-2-Amino-6-(*N*-propylamino)-4,5,6,7-tetrahydrobenzothiazole (9.15 g, 43.28 mmol) in 500 ml round-bottom flask was dissolved in 30 ml of ethanol and water (0.78 g, 43.33 mmol) was added. A solution was cooled in an ice bath to 0°C and gaseous HCl_{(g)}, was blown through whereby a white precipitate fell out. The roundbottomed flask was sealed and it was stirred over night at room temperature. The next day the precipitate was filtered off by suction and washed with a small amount of anhydrous ethanol. The precipitate was transferred into 100 ml round-bottom flask and anhydrous ethanol (50 ml) was added. The suspension was heated to 45°C and ethanol was evaporated on a rotatory evaporator. The process was repeated for another two times in order to drive out all of the excessive HCl_{(g)}. The product was recrystallized from methanol: a salt was dissolved in methanol (70 ml) at 45°C, approximately 40 ml of methanol was evaporated and 20 ml of ethanol were added. It was cooled to room temperature and the resulting precipitate was filtered by suction, washed with some cooled anhydrous ethanol and dried in vacuum over P₂O₅ and NaOH.
Yield: 11.631 g (89.01 %)

### Example 5

### Synthesis of N-(2-amino-4,5,6,7-tetrahydrobenzo[d]thiazole-6-yl)-2-nitrobenzenesulfonamide

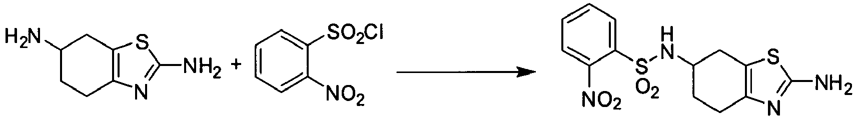

2-Nitrobenzenesulfonyl chloride (390 g, 1.76 mol) was dissolved in 4 1 of THF. The solution was cooled to approximately -10°C. Triethylamine (Et₃N) (740 g, 7.313 mol) and (6S)-4,5,6,7-tetrahydrobenzotiazole-2,6-diamine (327 g, 1.932 mol) were added. The suspension was heated during mixing to approximately 25°C and allowed to react at this temperature for approximately 1 hour.

Precipitated triethylammonium chloride (Et₃NH⁺Cl⁻) was filtered off. The filtrate was concentrated to about 1/3 of the volume and water (2 l) was added. Again, approximately 1/2 of the solvent was distilled off. Water (2 l) was added, the mixture was cooled to about 25°C and mixed for about 1 hour. The precipitated product ((6S)-*N*-(2-amino-4,5,6,7-tetrahydrobenzotiazole-6-yl)-2-nitrobenzenesulfonamide) was separated by filtration or centrifuging.

### Example 6

### Synthesis of (S)-(-)-2-Amino-6-(N-propylamino)-4,5,6,7-tetrahidrobenzo[d]thiazole dihydrochloride hydrate

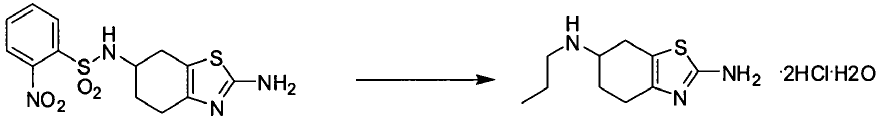

Potassium carbonate (1890 g, 13.675 mol), (6S)-*N*-(2-amino-4,5,6,7-tetrahydrobenzotiazole-6-yl)-2-nitrobenzenesulfonamide (590 g, 1.665 mol) and propyl bromide (1.09 1, 12 mol) were suspended in 4.1 1 of acetonitrile. The mixture was heated during stirring to approximately 60°C and mixed at this temperature for about 12 hours. The mixture was cooled to about 25°C. Potassium bromide was removed by filtration. The solution was concentrated to about 1/4 of the volume (not exceeding 60°C) and cooled to the room temperature. Methylene chloride (2 1) and 1 M NaOH water solution (2.43 1) were added and the mixture was mixed for about 30 minutes. Phases were separated and water phase was washed again with methylene chloride (1.46 1). Organic phases were collected and concentrated to about 1/10 of the volume. 0.83 1 of ethanol was added and the solution was concentrated to 1/10 of the volume. 3.35 1 of ethanol was added and ethanolic solution of (6S)-*N*-(2-amino-4,5,6,7-tetrahydrobenzotiazole-6-yl)-2-nitro-N-propylbenzenesulfonamide was stored for further reaction.

Ethanol (2.35 1) and of LiOH (288 g, 12 mol) were put into the reactor and the suspension was cooled to 0 - 5°C. During about 30 minutes thioglycolic acid (SHCH₂COOH) (720 g, 7.816 mol) was added (the temperature must not exceed 25°C). The suspension was heated to about 25°C and mixed for about 45 minutes. Ethanolic solution of (6S)-*N*-(2-amino-4,5,6,7-tetrahydrobenzotiazole-6-yl)-2-nitro-N-propylbenzenesulfonamide was added. The air in the reactor was replaced by nitrogen. The mixture was heated to about 50°C and mixed at this temperature for about 4 hours. The mixture was cooled to about 25°C and filtrated. The filtrate was concentrated at 40°C to about 1/4 of the volume and cooled to the ambient temperature. Methylene chloride (4.23 1) and of 1M aqueous NaOH solution solution (2.53 1) were added and the mixture was mixed for about 30 minutes. Phases were separated and water phase was washed again with 4.23 1 of methylene chloride. Organic phases containing pramipexole were collected and concentrated to about 1/4 of the volume. 5 1 of ethanol was added.

To the ethanolic solution of pramipexole water was added (27.6 ml,1.53 mol) and solution was cooled to about -10°C. Gaseous HCl_{(g)} was introduced into the solution (200 g). The temperature of the solution and later the suspension must not exceed 25°C during addition of gaseous HCl_{(g)}. After the addition the suspension was heated to about 40°C and concentrated to 2/3 of the volume. 2.65 1 of ethanol was added and the suspension was concentrated to 1/2 of the volume. Again 3.5 1 of ethanol was added and the suspension was concentrated to 1/2 of the volume. The solution was cooled to about -15°C and the product was separated by filtration. The product was dried at 25°C and finally at 40°C on air.

## Claims

1. A process for the preparation of pramipexole, **characterized in that** it comprises:
(i) a reaction of 4,5,6,7-tetrahydrobenzo[*d*]thiazole-2,6-diamine of formula I 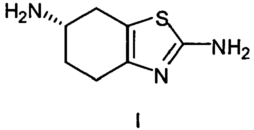 with nitro or halogen substituted or unsubstituted aryl sulfonyl chloride of formula II 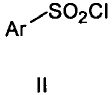 whereby sulfonamide intermediate of formula III 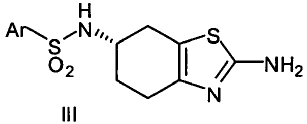 is obtained;
(ii) a reaction of an intermediate of formula III with a compound of formula IV, 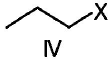 wherein X can be selected from Cl, Br, I, or other nucleophilically exchangeable group, such as OTs, OMs, whereby an intermediate of formula V 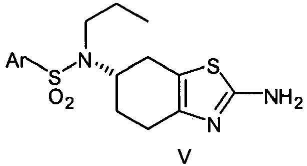 is obtained;
(iii) a conversion of an intermediate of formula V with a selective reagent for cleaving off the sulfonyl protection into pramipexole;
(iv) optional conversion of pramipexole into pharmaceutically acceptable salt.

2. A process according to claim 1, wherein a reaction between intermediates I and II is carried out in the presence of a base.

3. A process according to claim 1, wherein as nitro substituted aryl sulfonyl chloride of formula II, mono-, di- or tri-nitro substituted benzene sulfonyl chloride is used.

4. A process according to claim 1, wherein as nitro substituted aryl sulfonyl chloride of formula II, 2-nitrobenzene sulfonyl chloride is used.

5. A process according to claim 1, wherein a reaction between intermediate III and a compound of formula IV is carried out in the presence of a base.

6. A process according to claim 1, wherein as a selective reagent, thiol in the presence of a base or alkoxide is used.

7. A process according to claim 6, wherein as thiol, thiophenol or thioglycolic acid in the presence of a base is used.

8. A process according to claim 1, wherein the process is a continuous process.

9. A compound of formula III 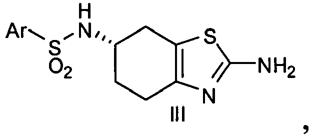 wherein Ar represents nitro or halo substituted aryl.

10. A compound of formula V 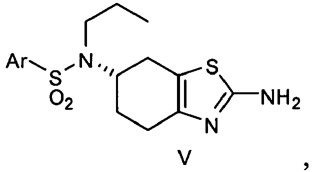 wherein Ar represents nitro or halo substitured aryl.

## Patentansprüche

1. Verfahren zur Herstellung von Pramipexol, **dadurch gekennzeichnet, dass** es Folgendes umfasst:
(i) eine Umsetzung von 4,5,6,7-Tetrahydrobenzo[d]thiazol-2,6-diamin der Formel I 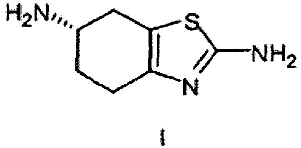 mit einem nitro- oder halogensubstituierten oder unsubstituierten Arylsulfonylchlorid der Formel II 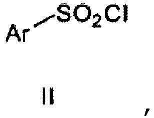 wodurch man das Sulfonamidzwischenprodukt der Formel III 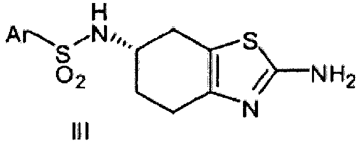 erhält,
(ii) eine Umsetzung eines Zwischenprodukts der Formel III mit einer Verbindung der Formel IV, 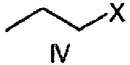 in welcher X aus Cl, Br, I oder einer anderen nukleophil austauschbaren Gruppe wie OTs oder OMs ausgewählt sein kann, wodurch man ein Zwischenprodukt der Formel V 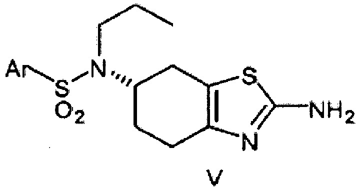 erhält,
(iii) einer Umwandlung eines Zwischenprodukts der Formel V mit einem selektiven Reagenz zur Abspaltung der Sulfonylschutzgruppe unter Erhalt von Pramipexol,
(iv) gegebenenfalls die Umwandlung von Pramipexol in ein pharmazeutisch unbedenkliches Salz.

2. Verfahren nach Anspruch 1, wobei man eine Umsetzung zwischen den Zwischenprodukten I und II in Gegenwart einer Base durchführt.

3. Verfahren nach Anspruch 1, wobei man als nitrosubstituiertes Arylsulfonylchlorid der Formel II mono-, di- oder trinitrosubstituiertes Benzolsulfonylchlorid verwendet.

4. Verfahren nach Anspruch 1, wobei man als nitrosubstituiertes Arylsulfonylchlorid der Formel II 2-Nitrobenzolsulfonylchlorid verwendet.

5. Verfahren nach Anspruch 1, wobei man eine Umsetzung zwischen Zwischenprodukt III und einer Verbindung der Formel IV in Gegenwart einer Base durchführt.

6. Verfahren nach Anspruch 1, wobei man als selektives Reagenz Thiol in Gegenwart einer Base oder eines Alkoholats verwendet.

7. Verfahren nach Anspruch 6, wobei man als Thiol Thiophenol oder Thioglykolsäure in Gegenwart einer Base verwendet.

8. Verfahren nach Anspruch 1, wobei es sich bei dem Verfahren um ein kontinuierliches Verfahren handelt.

9. Verbindung der Formel III 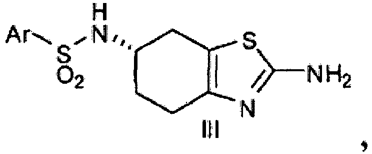 in welcher Ar für nitro- oder halogensubstituiertes Aryl steht.

10. Verbindung der Formel V 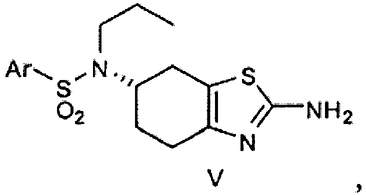 in welcher Ar für nitro- oder halogensubstituiertes Aryl steht.

## Revendications

1. Procédé de préparation de pramipexole, **caractérisé en ce qu'**il comprend :
(i) une réaction de 4,5,6,7-tétrahydrobenzo[*d*]thiazole-2,6-diamine de formule I 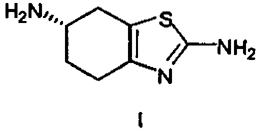 avec un chlorure d'arylsulfonyle substitué ou non substitué par nitro ou halogène de formule II 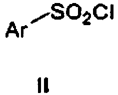 dans lequel le sulfonamide intermédiaire de formule III 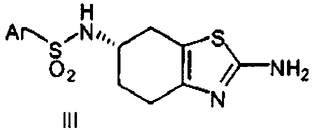 est obtenu ;
(ii) une réaction d'un intermédiaire de formule III avec un composé de formule IV, 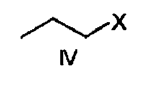 dans lequel X peut être choisi parmi Cl, Br, I, ou un autre groupe échangeable de façon nucléophile, tel que OTs, OMs, de sorte qu'un intermédiaire de formule V 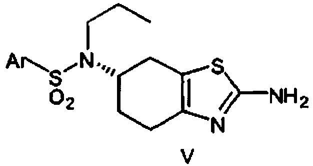 soit obtenu ;
(iii) une conversion d'un intermédiaire de formule V avec un réactif sélectif pour cliver la protection de sulfonyle en pramipexole ;
(iv) la conversion facultative de pramipexole en sel pharmaceutiquement acceptable.

2. Procédé selon la revendication 1, dans lequel une réaction entre les intermédiaires I et II est conduite en présence d'une base.

3. Procédé selon la revendication 1, dans lequel, en tant que chlorure d'arylsulfonyle substitué par nitro de formule II, un chlorure de benzènesulfonyle mono-, di- ou tri-nitro-substitué est utilisé.

4. Procédé selon la revendication 1, dans lequel, en tant que chlorure d'arylsulfonyle substitué par nitro de formule II, le chlorure de 2-nitrobenzènesulfonyle est utilisé.

5. Procédé selon la revendication 1, dans lequel une réaction entre l'intermédiaire III et un composé de formule IV est conduite en présence d'une base.

6. Procédé selon la revendication 1, dans lequel, en tant que réactif sélectif, un thiol en présence d'une base ou d'un alcoxyde est utilisé.

7. Procédé selon la revendication 6, dans lequel, en tant que thiol, le thiophénol ou l'acide thioglycolique en présence d'une base est utilisé.

8. Procédé selon la revendication 1, dans lequel le procédé est un procédé continu.

9. Composé de formule III 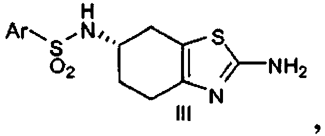 dans lequel Ar représente aryle substitué par nitro ou halogéno.

10. Composé de formule V 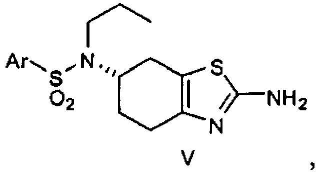 dans lequel Ar représente aryle substitué par nitro ou halogéno.
